(19) Europäisches Patentamt
European Patent Office
Office européen des brevets

(11) **EP 4 513 182 A1**

(12) **EUROPEAN PATENT APPLICATION**

(43) Date of publication:
**26.02.2025 Bulletin 2025/09**

(21) Application number: **23193283.1**

(22) Date of filing: **24.08.2023**

(51) International Patent Classification (IPC):
**G01N 27/327** (2006.01)   **A61J 1/10** (2006.01)
**A61J 1/18** (2023.01)   **B01L 3/00** (2006.01)
**G01N 33/543** (2006.01)

(52) Cooperative Patent Classification (CPC):
**G01N 27/3276; G01N 33/5438;** A61J 1/10;
A61J 2200/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Alekhmimi, Nuha Khalid
Al Olaya Riyadh, 12331 4624 (SA)**

(72) Inventor: **Alekhmimi, Nuha Khalid
Al Olaya Riyadh, 12331 4624 (SA)**

(74) Representative: **Hepp Wenger Ryffel AG
Friedtalweg 5
9500 Wil (CH)**

(54) **REDOX-FREE ELECTROCHEMICAL SENSOR**

(57)    The present invention refers to an electrochemical sensor (1) for use in a fluid container (11), preferably in a blood bag for blood analysis. The sensor (1) comprises two electrodes (4a, 4b) and a substrate (2) arranged between the two electrodes (4a, 4b). The substrate (2) has a conductive layer (3). A binding bi-omolecule (6, 7, 8, 9) is attached to the conductive layer (3) via a crosslinker (5). The invention also refers to a method of preparing such electrochemical sensor (1) and a fluid container (11) comprising at least one such electrochemical sensor (1).

FIG 1

**Description**

The present invention refers to an electrochemical sensor for use in fluid containers, to a method of preparing the electrochemical sensors and to a fluid container comprising the electrochemical sensor according to the preambles of the independent claims.

Blood banks are specialized huge stores for keeping blood bags with different groups, i.e.: blood groups (B, A, AB, O) with their Rhesus factor. Blood Banks distribution in the Kingdom of Saudi Arabia are increasing year after year in all regions of the Kingdom. The increasing number of blood banks unfortunately results in increasing medical errors as well, such as: wrong donation, the patient's blood is incompatible with the donor and may lead to kidney failure; blood transfusion from a donor with an infectious disease; and temperature of blood bags are not at 37 degrees centigrade, which may affect the patient's body with shock or sensitivity that may lead to death.

But blood bank medical errors are a health problem worldwide, not only the Kingdom of Saudi Arabia. There is an urgent need to reduce medical problems occurring with blood transfusion.

US 10,285,906 B2 describes blood bags with RFID-tags connected to the bags to avoid problems occurring with bar code labeling and thus ensuring secure tracking of the blood container.

US 2021/0146026 A1 discloses a bag for storing biological substances. An electronic device is attached to the inner wall of the bag. The electronic device comprises a sensor for measuring a physiological parameter, such as temperature, pH, conductivity, glucose, $O_2$, $CO_2$; or physical parameter of the biological substance. A radiofrequency device is communicably coupled to the sensor.

However, there is a need for a bag for biological substances that allows a rapid identification of the biological sample itself and pathogenic factor associated with the sample. There is also a need for correct storage of these data on the bag. In addition, there is a need for a direct analysis of the sample to avoid incorrect labeling due to wrong allocation of the analysis to the sample.

In particular, there is a need for rapid biosensing techniques for blood analysis that are low in cost and where data can be stored in an easy manner.

It is thus an objective of the present invention to overcome the drawbacks of the prior art. In particular, it is an objective of the present invention to provide a rapid testing sensor for biological samples. It is further an objective of the present invention to provide an array with a plurality of such sensors and a fluid container with such a sensor or array. It is even further an objective of the present invention to provide a method for preparing such sensor.

The objectives are solved by the subject-matter of the independent claims. Preferred embodiments are described with regard to the dependent claims.

A first aspect of the invention refers to an electrochemical sensor for use in a fluid container, preferably in a blood bag. The sensor comprises two electrodes and a substrate arranged between the two electrodes. The substrate has a conductive layer. A binding biomolecule is attached to the conductive layer via a crosslinker.

The electrodes can be made of gold, indium tin oxide, copper and the like. The electrodes can be made of the same material as the conductive layer.

The sensor comprises preferably only two electrodes, in particular a sensing electrode and a counter electrode. The attachment of the biomolecule to the conductive layer can be physical or chemical. The attachment can result from covalent linkage or through van-der-Waals forces or H-bridges. The crosslinker preferably immobilizes the binding biomolecule on the conductive layer.

The counter electrode preferably provides a contact with the analyte or sample solution so that current can be applied to the sensing electrode. The sensing electrode serves as a functionalizing element to build layers for the biochemical binding reaction. Upon binding of an analyte molecule of the sample to the binding biomolecule, a change in, for example, the capacitance can be detected with a potentiostat known in the art, such as Gamry potentiostat 1000E. The electrochemical sensor according to the invention is easy to manufacture. Since its detection mechanism is not based on redox chemistry, only two electrodes are needed which makes the more cost-effective than known sensors in the art.

The binding biomolecule is preferably selected from the group consisting of: peptides; proteins, in particular antibodies and enzymes; DNA sequences; oligonucleotides; RNA sequences; or cells.

In particular, the binding biomolecule can be selected from the group consisting of: metalloproteinase, neutrophile elastase, blood antibodies, aptamers for HIV 1 or 2, HBSC (hepatitis C), B (hepatitis B), A (hepatitis A), human T-lymphotropic virus.

The binding biomolecule can also be part of a nucleic acid tests for plasmodium parasites causing malaria; T-cruzi; West Nile Virus, Cytomegalovirus (CMV).

The conductive layer can be a metal-containing layer, the metal being preferably selected from the group consisting of gold, platinum, copper, aluminium, cobalt, silver or indium tin oxide; or the conductive layer is an indium tin oxide, a carbon or a silicon layer.

In general, any conductive material is possible.

The substrate can be any material suitable to be coated with a conductive layer. The use of aluminium is preferred. Aluminium is relatively cheap compared to gold or platin sensor known in the art.

Most preferably the sensor can comprise an aluminium substrate coated with indium tin oxide.

Advantageously, the crosslinker is derived from a sulphur-containing compound comprising at least one additional functional group. The functional group is preferably selected from the group consisting of: aldehyde, carboxyl, hydroxyl or amine group.

The crosslinker may also be an aldehyde, hydroxyl compound or amine with or without sulphur. The nature of the crosslinker strongly dependent on the nature of the attachable biomolecule and the surface the biomolecule is attached to. For example, sulphur-containing compounds such as L-cysteine, mercaptoethanol or cysteamine have a high affinity to gold.

Most preferred are crosslinkers derived from aminothiols, such as cysteamine; sulphur-containing amino acids, such as cysteine; dialdehyde, such as glutaraldehyde; graphene and biotin.

Advantageously, the sensor is connected to or connectable to a reading device, preferably a potentiostat. The data measured with the reading device may be transferred to a processing unit, preferably a computer.

Advantageously, the sensor can also be connected to or be connectable to a processing unit, preferably a computer. Any data obtained with the sensor can be transferred and stored to the processing unit. A computer-implemented software may transfer the data to a visual output allowing a fast analysis and/or diagnosis of the sample.

The sensor can be connected or connectable to a data storage unit, preferably an RFID tag. The data transmission can be wireless. The transmission and storage can work by using a tracking system, such as GPS.

A second aspect of the invention refers to an array comprising a plurality of electrochemical sensors as previously described. Each electrochemical sensor is equipped with a different biomolecule or different biomolecules for detecting different pathogens or biological factors associated with the sample.

The array allows the detection of different sample related analytes simultaneously. The array can be realized as a microchip.

A third aspect of the invention relates to a method of preparing an electrochemical sensor, preferably an electrochemical sensor as previously described, more preferably an electrochemical sensor for a blood bag. The method comprises the steps of:

> a) Providing a substrate with two electrodes, the substrate being preferably arrange between the two electrodes,
> b) Coating the substrate with a conductive layer,
> c) Providing at least a first crosslinking agent having at least a first functional group and a second functional group,
> d) Reacting the conductive layer with the first functional group of the first crosslinking agent,
> e) Attaching a binding biomolecule to the second functional group of the first crosslinking agent.

Step e) can be a physical or chemical attachment resulting from covalent, van-der-Waals or H-bridge bonding. The word "attaching" can comprise a direct binding to the crosslinking agent or an indirect binding via additional molecules.

Preferably, after step c) the second functional group of the first crosslinking agent is reacted with a second crosslinking agent, the binding biomolecule being then attached to the second crosslinking agent. The second crosslinking agent has preferably at least two reactive sites to attach to the first crosslinking agent and to allow attachment of the binding biomolecule. The attachment is preferably a chemical bond.

Advantageously, the first crosslinking agent is a sulphur-containing compound comprising at least one additional functional group. The sulphur-containing compound is preferably selected from the group consisting of: L-cysteine, cystamine, biotin, aldehyde, carboxyl compound, hydroxyl compound or amine.

The second crosslinking agent is preferably selected from the group consisting of: dialdehyde, preferably glutaraldehyde; carboxyl, amine, graphene, biotin.

The biomolecule can be selected from the group consisting of: peptides; proteins, in particular antibodies and enzymes; DNA sequences; oligonucleotides; RNA sequences; or cells.

Another aspect of the invention refers to a method of preparing an array comprising several electrochemical sensors as previously described. The method comprises the steps of joining several electrochemical sensor to an array, in particular in the form of a microchip.

A fourth aspect of the invention refers to a fluid container, preferably for biological samples, most preferably a blood bag, comprising at least one electrochemical sensor as previously described.

Most preferably, the blood bag comprises several electrochemical sensors, even more preferably an array of several electrochemical sensors, for detecting different parameters allowing a rapid and almost complete analysis of the biological sample inside the bag.

The fluid container may comprise a main compartment and a side compartment. The main compartment is preferably used to collect and store the main volume of the sample, preferably for re-use, while a smaller volume of the sample can be collected and stored in the side compartment for analysis.

The electrochemical sensor or the array can be arranged in the side compartment of the fluid container. For example, in a blood bag, a first small volume of approximately 50 mL of the blood sample is directed to the side compartment during blood collection and analysed while the main volume of approximately 450 mL of the blood collection is directed to main compartment.

Alternatively, the fluid container may comprise a main compartment and the electrochemical sensor or array can be directly located in the main compartment.

It is one advantage of the present invention, that the sample can be directly analysed when collected. Thus, it is guaranteed that potentially relevant pathogens can be detected without becoming inactive due to storage time and associated temperature differences or other factors.

Further, since the compartment of analysis and the sample compartment are arranged in the same bag, errors due to incorrect sample allocation are prevented. This is in particular relevant with regard to blood donation.

Each sensor can be connected to a reading device for analysis. The reading device can be a potentiostat. The potentiostat can be connected or can be connectable to a processing unit as described above.

The bag can comprise a data storage unit, preferably an RFID tag, for storing information on the fluid stored in the container. The storage unit is preferably connected or connectable to the at least one electrochemical sensor and/or to the processing unit.

Advantageously, the data storage unit is connected or connectable to a processing unit, preferably a computer.

A further aspect of the invention is directed to a method of collecting and analysing a sample, preferably a biological sample and even more preferably blood from a blood donor. The method comprises the steps of:

- Extracting the sample, preferably from a blood vessel,
- Directing the sample to a fluid container as previously described,
- Collecting a first volume of the sample in the side compartment
- Collecting the main volume of the sample in the main compartment,
- subjecting the sample in the side compartment to an electrochemical sensor or array as previously describes for analysis,
- optionally transferring the data obtained with the electrochemical sensor or array to a processing unit, preferably a computer, to process the data into a readable format,
- optionally transferring the processed data to a data storage unit, preferably an RFID tag on the fluid container.

The method allows a rapid analysis of the sample directly after sample extraction. The data can be directly stored on the fluid bag, avoiding errors due to incorrect sample allocation.

The invention will be explained in more details based on the following figures and examples. The invention is not limited to these embodiments. It shows:

Figure 1    A schematic representation of the working principle of a sensor according to the invention
Figure 2    A preparation method for a first electrochemical sensor according to the invention.
Figure 3    A preparation method for a second electrochemical sensor according to the invention.
Figure 4    A fluid container according to the invention.
Figure 5    The fluid container of figure 4 connected to a computer.
Figure 6    Bode plot of positive sample (A, MMP-9 in blood) and negative sample (B, without MMP-9).
Figure 7    Change in capacitance for the plot in figure 6.
Figure 8    The average change in capacitance against positive and negative samples.
Figure 9    The result of a non-faradaic detection of MMP-2 in blood or broncho aleveolar lavage (BAL).

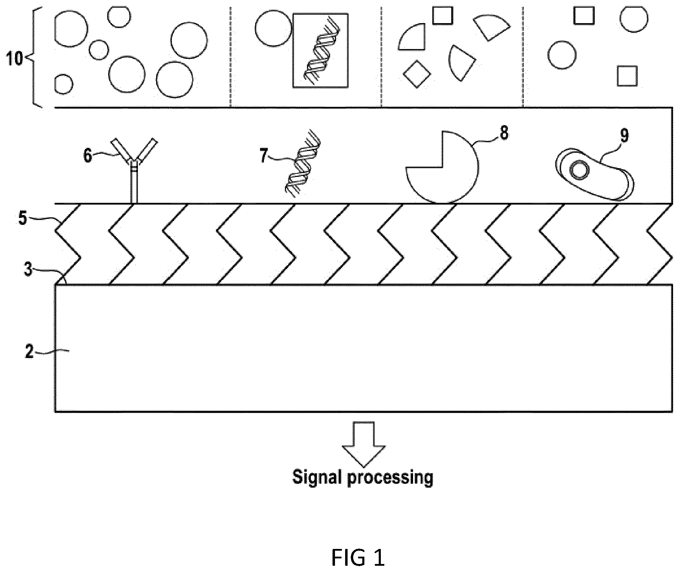

Figure 1 shows a schematic representation of the working principles of a sensor according to the invention. A substrate 2 is provided with a conductive layer 3 to which one or more crosslinking molecule 5 can be attached. To the one or more crosslinking molecule one or more binding biomolecules can be attached such as an antibody 6, a DNA sequence 7, enzymes 8 or cells 9. The sensor can be subjected to a biological sample such as blood where it can detect a variety of pathogens or other molecule being specific for the samples. Such factors 10 can be virus-DNA, proteins, enzymes and the like. The sensor can be connected to a reading device as indicated with the arrow to "signal processing".

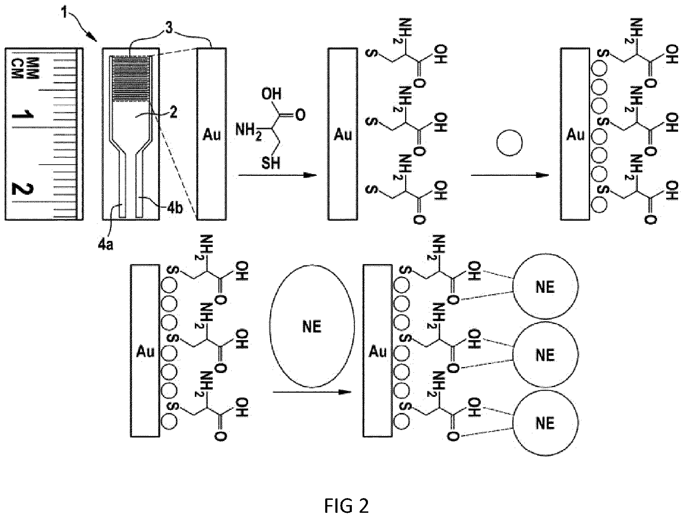

Figure 2 shows one specific method of providing a sensor 1 with a neutrophile elastase (NE) as binding biomolecule. A substrate 2 is coated with a gold layer 3 between two electrodes 4a and 4b. In a next step, the gold surface is modified with l-cysteine as crosslinking agent. In order to block further reactions, 2-mercaptoethanol (circle) is added, blocking the gold surfaces. The preparation of the sensor is followed by a step for adding the neutrophile elastase (NE), forming hydrogen bridges with the free carboxylic acid groups of the cysteine, attached to the gold surface. This immobilizes the neutrophile elastase as binding biomolecule on the surface of the sensor. Neutrophile elastase is a serine proteinase and has a broad substrate specificity. Neutrophile elastase is secreted by neutrophils during inflammation.

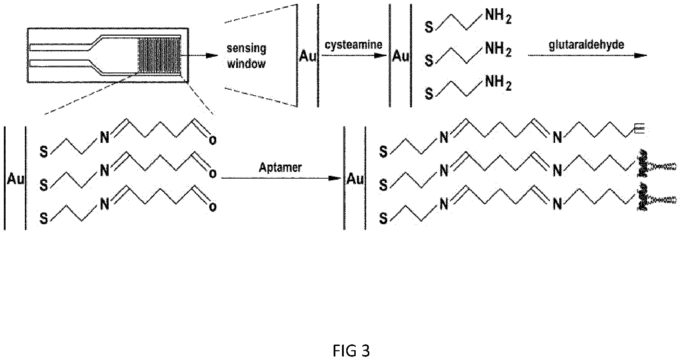

Figure 3 shows a preparation method for a second electrochemical sensor according to the invention. The substrate is again arranged between two electrodes and at least partially coated with a gold layer. The gold layer was then subjected to cysteamine to attach the cysteamine with the thiol-group to the gold surface. In a subsequent step, the cysteamine was reacted with a second crosslinking agent, glutaraldehyde. The free aldehyde group of the attached glutaraldehyde allowed the reaction with an aptamer as binding biomolecule. Aptamers are artificial oligonucleotides that can bind one or more specific target molecules. It is also possible to attach different aptamers to allow detection of different proteins in the sample. For example, the aptamer can be configured to detect one or more of the following viruses: HIV 1 or 2, HBSC, B, A, human T-lymphotropic virus anti-HTLV I/II.

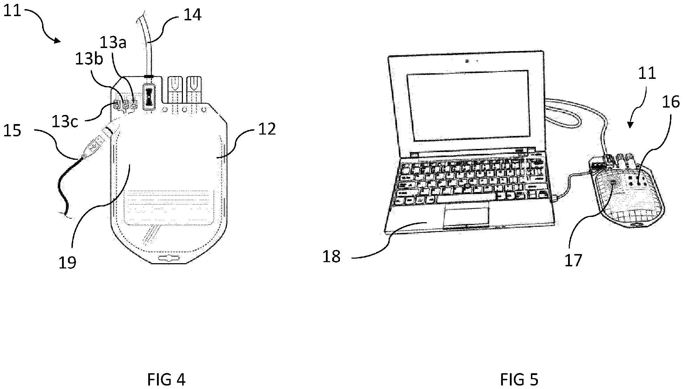

Figure 4 shows a fluid container according to the invention. The fluid container is a blood bag 11 comprising a sterile plastic bag 12 with a main compartment 19. The plastic bag 12 is equipped with 3 chips 13a, 13b, 13c, each chip 13a, 13b, 13c comprising up to for electrochemical sensors. The first biosensor chip 13a contains sensors for detecting a type of infection by electrochemical sensors coated with binding biomolecule, e.g. protein, peptides or DNA sequences recognizing microbes causing the infection. A second chip 13b is equipped with electrochemical sensors to recognize a compatibility or incompatibility between blood donor and acceptor. A third chip 13c is equipped with electrochemical sensors to determine the blood group of the patient. The chips are attached to a reading output 14, connectable to a reading device. The bag 11 also comprises an USB port 15 for transferring and processing collected data from the biosensor chips 13a, 13b, 13c to a processing unit.

Figure 5 shows the blood bag 11 of figure 4 connected to a computer 18 as processing unit. Further, the blood bag is equipped with an RFID tag 17 and a sensor 16 for measuring the temperature of the blood bag.

EXAMPLES

Example 1

The preparation of the sensor as shown in figure 2 is described below:
Firstly, the gold layer (sensing window) of Au-ITO (indium tin oxide)s was incubated with an ethanolic solution of 1 mM cysteamine for one hour in darkness at room temperature. The sensing window was then rinsed with ethanol, followed by blown-drying with nitrogen. Then, the sensing window was incubated with 50 $\mu$L of 2.5% glutaraldehyde in PBS for one hour. This was followed by rinsing the sensing window with deionized water and blow-drying with nitrogen. Proteases, pathogen, Blood grouping and cross matching detection - antibodies were then attached on the surface of the sensing window by incubating the sensing window with 50 $\mu$L of 1 $\mu$g/mL MMP-9 or MMP-2 (matrix metalloproteinases) and Elastase-antibodies in deionized water for one hour. This provides a multiplex-sensing opportunity in one sensor. The elastase is another protease induced secretion from neutrophils immune cells.

Rinsing with deionized water was then carried out. Finally, blocking was performed by introducing 5% of 50 $\mu$L BSA in PBS for 30 minutes.

The redox-free biosensor measurements was carried out using a small reader GAMRY (interface 1000™) potentiostat. Prior to proteases, pathogen, Blood grouping and cross matching detection, the baseline was measured as followed: The sensing window was introduced with 50 $\mu$L PBS. Then, open circuit potential (OCP) measurement was carried out for 100 seconds, followed by EIS measurements in the frequency range 200 mHz to 100 kHz with an applied sinusoidal perturbation of 10 mV. EIS measurements was repeated three times until a stable baseline resulted. After extracting the baseline, the PBS droplet was rinsed with deionized water followed by nitrogen blow-drying. Different concentrations of blood-antigen in PBS were then introduced onto the sensing window (from 500 pg/mL to 5 $\mu$g/mL) followed by EIS measurements. Rinsing with deionized water was performed after each blood patients-antigen concentration incubation. Each concentration incubation lasted for 5 minutes and changes in capacitance were normalized to the baseline of the biosensor. Similar EIS measurements were performed against successive PBS (blank) incubations. These steps can be used to evaluate the biosensor performance (i.e. sensitivity and limit of detection (LoD)) of the biosensor. Changes in capacitance were normalized to the baseline of the biosensor. All experiments were repeated three times against blood sample-antigen and against the blank samples. Another important factor of the biosensors is selectivity, the biosensors were tested against positives samples of a patient containing a mixture of MMP-2/MMP-9-seq-Peptide, pepsin and E-coli. Moreover, the biosensors were tested against negative samples which contain clean blood sample of blood culture without any bacterial or virus - control.

*Nonstop Monitoring*

EIS (electrical impedance spectroscopy) runs the experiments within a wide range of frequency. It takes around 3 minutes to run a complete EIS cycle if the frequency range is set to 200 mHz to 100 kHz. In order to perform EIS continuous monitoring, the EIS cycle has to be limited to only one single frequency to speed-up the measurement. In the present invention, maximum capacitive changes upon MMP-9/MMP-2-ABTAMER/blood-antigen binding were observed at 49.7 Hz. Therefore, for the continuous monitoring of proteases, we narrowed the frequency range to 49.7 Hz. Measurements were performed with an applied sinusoidal perturbation of 10 mV. Firstly, the sensing window was introduced with 50 µL PBS, then EIS measurements at only 49.7 Hz were carried out. The next EIS cycle, 500 pg/mL of proteases were added into the PBS droplet and EIS cycles were repeated until a significant change in capacitance was determined. Then, 5 ng/mL MMP-9 was added to the PBS droplet and EIS measurements were repeated until a significant change in capacitance was observed. The same procedure was repeated for all blood samples of any protease concentration of 50 ng/mL, 500 ng/mL and 5 µg/mL. Each EIS cycle lasted for around 15 seconds. No further injections of MMP-2/MMP-9 were performed since the sensor saturated at 5 ug/mL MMP9/MMP-2. Similar experiment was carried out against only PBS without injecting any molecules to observe the changes in capacitance against blank samples and to understand the behavior of the biosensors.

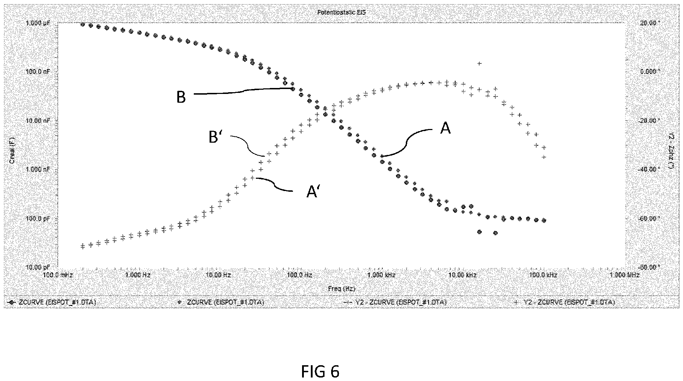

Figure 6 shows a Bode plot of positive (A; MMP-9 in blood) and negative samples (B, without MMP-9). As can be seen there are two dotted lines and two star-like lines. Two of them correspond to Creal (dotted; A,B) (or capacitance) and the other two (star-like; A',B') correspond to Zphz (or phase) according to the description of the axes. The Bode plot represent signals, capacitance or phase against frequency. Frequency increases from left to right on the X-axis. The region of interest is below 100 Hz. Above 100 Hz the signals correspond to instruments and surrounding environment. Around 49.7 Hz, line A is greater in capacitance than line B. Only one frequency point is chosen. This resembles the one specific frequency the device according to the invention is able to detect. It will not be necessary to detect a wide frequency range when the device is in use. So, 49.7 Hz was chosen as the point where there is the largest change in capacitance between the positive and negative samples.

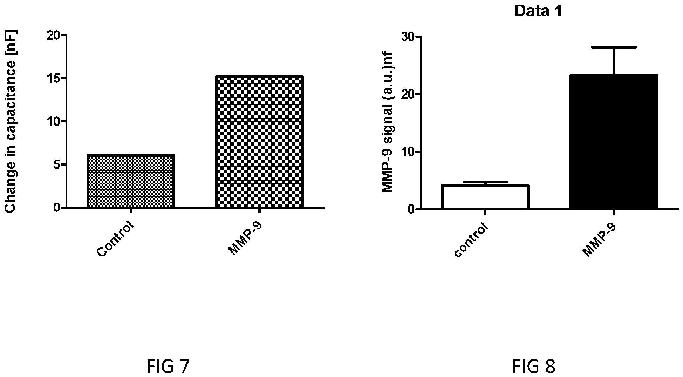

Figure 7 shows the change in capacitance at around 30 Hz for the positive sample (MMP-9) and negative (control) sample. The change in capacitance was calculated using the following equation:

```
Change in capacitance = control or positive sample capaci-
tance at certain frequency - capacitance of baseline at that
specific frequency.
```

The change in capacitance against the positive sample as seen in the bar chart of figure 7 is 15.17 nF, whereas the change in capacitance against the negative sample is 6.1 nF. The experiments were repeated three times and the average change in capacitance against positive and negative samples are shown in figure 8.

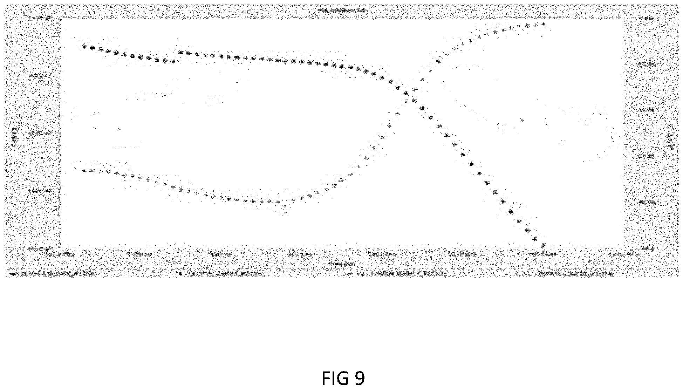

Figure 9 shows the result of a non-faradaic detection of MMP-2 in blood or BAL. Au-ITO were functionalized with cysteamine, glutaraldehyde and then MMP-2 antibodies as described with regard to Example 1.

Change in capacitance was observed at 2.516 Hz in this case. Figure 9 shows the Bode plot for sensors tested against positive (MMP2) and negative samples. Most of the data in figure 9 are on top of each other and the change in capacitance is at the nanoscale so they cannot be differentiated easily. Nevertheless, the change in capacitance against the positive samples is greater than against the negative (control sample). The change in capacitance is around 16.2 nF for MMP2 and 6.1 nF for the blank (negative) sample which indicates that the sensor was able to detect MMP2.

The sensor has responded against MMP2 but in a different manner compared to MMP-9. The sensors respond to different biomarkers at different frequencies.

**Claims**

1. Electrochemical sensor (1) for use in a fluid container (11), preferably in a blood bag for blood analysis, comprising two electrodes (4a, 4b) and a substrate (2) arranged between the two electrodes (4a, 4b), the substrate (2) having a conductive layer (3), **characterized in that** a binding biomolecule (6, 7, 8, 9) is attached to the conductive layer (3) via a crosslinker (5).

2. Electrochemical sensor (1) according to claim 1, wherein the binding biomolecule (6, 7, 8, 9) is selected from the group consisting of: peptides; proteins, in particular antibodies and enzymes; DNA sequences; oligonucleotides; RNA sequences; or cells.

3. Electrochemical sensor (1) according to one of the previous claims, wherein the binding biomolecule (6, 7, 8, 9) is selected from the group consisting of: metalloproteinase, neutrophile elastase (NE), blood antibodies, aptamers for HIV 1 or 2, HBSC, B, A, human T-lymphotropic virus.

4. Electrochemical sensor (1) according to one of the previous claims, wherein the conductive layer (3) is a metal-containing layer, the metal being preferably selected from the group consisting of gold, platinum, copper, aluminum, cobalt, silver or indium tin oxide; or the conductive layer is a carbon or silicon layer.

5. Electrochemical sensor (1) according to one of the previous claims, wherein the crosslinker (5) is derived from a sulphur-containing compound comprising at least one additional functional group, wherein the functional group is preferably selected from the group consisting of: aldehyde, carboxyl, hydroxyl or amine group.

6. Electrochemical sensor (1) according to one of the previous claims wherein the sensor (1) is connected to or connectable to a processing unit, preferably a computer (18).

7. Electrochemical sensor (1) according to one of the previous claims, wherein the sensor (1) is connected or connectable to a data storage unit, preferably an RFID tag (17).

8. An array comprising a plurality of electrochemical sensors according to one of claims 1 to 7 each electrochemical sensor being equipped with different binding biomolecules.

9. Method of preparing an electrochemical sensor (1), preferably an electrochemical sensor (1) according to claim 1 to 7, more preferably an electrochemical sensor (1) for a blood bag, the method comprising the steps of:

   a) Providing a substrate (2) with two electrodes (4a, 4b), the substrate being preferably arranged between the two electrodes,
   b) Coating the substrate (2) with a conductive layer (3),
   c) Providing at least a first crosslinking agent (5) having at least a first functional group and a second functional group,
   d) Reacting the conductive layer (3) with the first functional group of the first crosslinking agent (5),
   e) Attaching a binding biomolecule (6, 7, 8, 9) to the second functional group of the first crosslinking agent (5).

10. Method according to claim 9, wherein after step c) the second functional group of the first crosslinking agent (5) is reacted with a second crosslinking agent after step c) and wherein the binding biomolecule (6, 7, 8, 9) is attached to the second crosslinking agent (5).

11. Method according to claim 9 or 10, wherein the first crosslinking agent (5) is a sulphur-containing compound comprising at least one additional functional group, the sulphur-containing compound preferably being selected from the group of: L-cysteine, cystamine, biotin, aldehyde, carboxyl compound, hydroxyl compound, or amine.

12. Method according to claim 10 or 11, wherein the second crosslinking agent is selected from the group consisting of: dialdehyde, preferably glutaraldehyde; carboxyl, amine, graphene, bioten.

13. Method according to one of claims 9 to 12, wherein the biomolecule (6, 7, 8, 9) is selected from the group consisting of: peptides; proteins, in particular antibodies and enzymes; DNA sequences; oligonucleotides; RNA sequences; or cells.

14. Fluid container (11), preferably for biological samples, most preferably a blood bag, comprising at least one electrochemical sensor (1) according to one of claims 1 to 7 or an array according to claim 8.

15. Fluid container (11) according to claim 14, wherein the bag comprises a data storage unit, preferably an RFID tag (17), for storing information on the fluid stored in the container (11), wherein storage unit is preferably connected or connectable to the at least one electrochemical sensor (1) or array.

16. Fluid container (11) according to claim 15, wherein the data storage unit is connected or connectable to a processing unit, preferably a computer (18).

FIG 1

FIG 2

FIG 3

FIG 4

FIG 5

FIG 6

FIG 7

FIG 8

FIG 9

Europäisches
Patentamt

European
Patent Office

Office européen
des brevets

# EUROPEAN SEARCH REPORT

Application Number

EP 23 19 3283

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ASSAIFAN ABDULAZIZ K ET AL: "Detection and Real-Time Monitoring of LDL-Cholesterol by Redox-Free Impedimetric Biosensors", BIOCHIP JOURNAL, KOREAN BIOCHIP SOC, SEOUL, SOUTH KOREA, vol. 16, no. 2, 6 May 2022 (2022-05-06), pages 197-206, XP037897391, ISSN: 1976-0280, DOI: 10.1007/S13206-022-00058-Z [retrieved on 2022-05-06] | 1,2,4-7, 9-13 | INV. G01N27/327 A61J1/10 A61J1/18 B01L3/00 G01N33/543 |
| Y | * Sections: Experimental details; figure 1 * | 3,8, 14-16 | |
| Y | US 6 340 588 B1 (NOVA MICHAEL P [US] ET AL) 22 January 2002 (2002-01-22) * figures 64-66; examples 12-13 * | 3,8, 14-16 | |
| A | US 2021/146026 A1 (SHAVIT MENACHEM [US]) 20 May 2021 (2021-05-20) * paragraphs [0097] - [0150]; figures 1-6 * | 14-16 | |
| A | US 2017/265789 A1 (NASERI SARA [US] ET AL) 21 September 2017 (2017-09-21) * paragraphs [0008] - [0010], [0022] - [0028], [0031] - [0033], [0062]; figures 2,13,14-17 * | 14-16 | |

| TECHNICAL FIELDS SEARCHED (IPC) |
|---|
| G01N B01L A61J |

The present search report has been drawn up for all claims

| Place of search | Date of completion of the search | Examiner |
|---|---|---|
| Munich | 5 February 2024 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 19 3283

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2024

| Patent document cited in search report | | | Publication date | Patent family member(s) | | | Publication date |
|---|---|---|---|---|---|---|---|
| US 6340588 | B1 | | 22-01-2002 | NONE | | | |
| US 2021146026 | A1 | | 20-05-2021 | CA | 2957526 | A1 | 11-02-2016 |
| | | | | CA | 3187726 | A1 | 11-02-2016 |
| | | | | CN | 106999347 | A | 01-08-2017 |
| | | | | EP | 3177258 | A1 | 14-06-2017 |
| | | | | US | 2017239404 | A1 | 24-08-2017 |
| | | | | US | 2019151519 | A1 | 23-05-2019 |
| | | | | US | 2021146026 | A1 | 20-05-2021 |
| | | | | WO | 2016023034 | A1 | 11-02-2016 |
| US 2017265789 | A1 | | 21-09-2017 | AU | 2015303880 | A1 | 16-03-2017 |
| | | | | CA | 2958554 | A1 | 25-02-2016 |
| | | | | CN | 106999164 | A | 01-08-2017 |
| | | | | DK | 3182901 | T3 | 16-01-2023 |
| | | | | EP | 3182901 | A1 | 28-06-2017 |
| | | | | JP | 6656249 | B2 | 04-03-2020 |
| | | | | JP | 2017532576 | A | 02-11-2017 |
| | | | | US | 2017265789 | A1 | 21-09-2017 |
| | | | | WO | 2016028497 | A1 | 25-02-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10285906 B2
- US 20210146026 A1